# EUROPÄISCHE PATENTSCHRIFT

(11) **EP 3 277 221 B1**
(45) Veröffentlichungstag und Bekanntmachung des Hinweises auf die Patenterteilung: **15.02.2023**
(21) Anmeldenummer: 16721350.3
(22) Anmeldetag: 23.03.2016
(51) Int. Cl.: A61F 2/24

(54) **HERZKLAPPENPROTHESE**
PROSTHETIC HEART VALVE
PROTHÈSE VALVULAIRE CARDIAQUE

(30) Priorität: 02.04.2015 DE 102015206099
(43) Veröffentlichungstag der Anmeldung: 07.02.2018
(73) Patentinhaber: Sievers, Hans-Hinrich, 24119 Kronshagen (DE)
(72) Erfinder: SIEVERS, Hans-Hinrich, 24119 Kronshagen (DE); SCHARFSCHWERDT, Michael, 23564 Lübeck (DE); HOF, Andreas, 23568 Lübeck (DE)
(74) Vertreter: Patentanwälte Vollmann Hemmer Lindfeld Partnerschaft mbB
(86) Internationale Anmeldenummer: PCT/DE2016/200151
(87) Internationale Veröffentlichungsnummer: WO 2016/155730

(56) Entgegenhaltungen:
- US-A1- 2009 264 989
- US-A1- 2010 076 549
- US-A1- 2011 022 157
- US-A1- 2013 274 872
- US-A1- 2014 188 219

## Beschreibung

Die Erfindung betrifft eine Herzklappenprothese, insbesondere eine Herzklappenprothese zum Ersatz der Aortenklappe.

Beispielsweise aus DE 10 2010 051 632 ist eine biologische Herzklappenprothese zum Ersatz der Aortenklappe bekannt. Bei derartigen biologischen Herzklappenprothesen besteht das Problem, dass sie verschleißen und gegebenenfalls ersetzt werden müssen.

US 2014/0188219 A1 offenbart eine Herzklappenprothese mit einem erweiterbaren Klappengestell. Das Klappengestell weist einen integralen Tragring auf, welcher im Befestigungsbereich der Herzklappenprothese angeordnet ist und welcher durch Krafteinwirkung aufweitbar ist.

US 2009/264989 A1 offenbart eine Herzklappenprothese mit einem Klappengestell, welches in den Bereichen zwischen den Kommissuren in Umfangsrichtung aufweitbar ist.

Dokument US2011022157 A1 offenbart eine Herzklappenprothese mit einem selbstexpandierbaren Stent ohne Solldehnungsstellen.

Nachteilig bei den bekannten Herzklappenprothesen ist, dass die Aufweitung des Befestigungsbereiches und des Klappengestells nur gleichzeitig erfolgen können, sodass es schwierig ist, die Herzklappenprothese an verschiedene anatomische Gegebenheiten ohne Funktionsbeeinträchtigung anzupassen.

Im Hinblick auf diese Problematik ist es Aufgabe der Erfindung, eine Herzklappenprothese dahingehend zu verbessern, dass sie zum einen auf einfache Weise durch eine weitere Herzklappenprothese ersetzt werden kann und zum anderen besser an die Anatomie eines Patienten anpassbar ist sowie stets eine zuverlässige Funktion der Klappensegel sicherstellt.

Diese Aufgabe wird durch eine Herzklappenprothese mit den in Anspruch 1 angegebenen Merkmalen gelöst. Bevorzugte Ausführungsformen ergeben sich aus den Unteransprüchen, der Beschreibung sowie den Figuren.

Die erfindungsgemäße Herzklappenprothese weist ein Klappengestell auf, an welchem mehrere Klappensegel befestigt sind. Vorzugsweise handelt es sich dabei um Klappensegel aus biologischem Material, das heißt es handelt sich um eine biologische Herzklappenprothese. Erfindungsgemäß sind drei Klappensegel vorgesehen und das Klappengestell ist entsprechend mit drei bogenförmigen Halteelementen geformt, welche in drei Kommissuren auslaufen und die Klappensegel tragen. Das Klappengestell ist vorzugsweise aus Metall ausgebildet und in einem gewissen Maß elastisch verformbar.

Axial, das heißt in Strömungsrichtung bzw. dieser entgegengerichtet an das Klappengestell anschließend, ist ein Befestigungsbereich zur Befestigung in einem Blutgefäß ausgebildet. In diesem kann insbesondere ein Nahtring gelegen sein, an welchem die Herzklappenprothese mit einem umgebenden Blutgefäß vernäht wird. In dem Befestigungsbereich ist ein Stabilisierungsring angeordnet, welcher eine vorgegebene Form und einen vorgegebenen Durchmesser des Befestigungsbereiches definiert. Insbesondere definiert dieser Stabilisierungsring vorzugsweise den maximalen Strömungsquerschnitt der Herzklappenprothese. Der Stabilisierungsring dient dazu, das Blutgefäß gegebenenfalls aufzuweiten, so dass der maximale Strömungsquerschnitt tatsächlich erreicht wird.

Erfindungsgemäß weist der Stabilisierungsring zumindest eine Solldehnungsstelle auf, welche es ermöglicht, den Stabilisierungsring durch eine radiale Krafteinwirkung, welche von innen auf seinen Innenumfang wirkt, aufzuweiten. Die Solldehnungsstelle ist so ausgebildet, dass sie sich vorzugsweise über die gesamte axiale Länge des Stabilisierungsringes erstreckt, so dass dieser sich bei Krafteinwirkung in Umfangsrichtung erweitern kann.

Diese Eigenschaft des Stabilisierungsringes kann dazu genutzt werden, die Herzklappenprothese bei Verschleiß durch eine neue Herzklappenprothese zu ersetzen, indem diese in das Innere der erfindungsgemäßen Herzklappenprothese eingeführt wird. Dies kann beispielsweise eine Transkatheter-Herzklappenprothese sein, welche über einen Katheter in das Gefäß und das Innere der bestehenden Herzklappenprothese eingebracht wird. Dort wird diese neue Herzklappenprothese dann erweitert, wobei die beschriebene Kraft auf den Innenumfang des Stabilisierungsringes der erfindungsgemäßen Herzklappenprothese wirkt, so dass der Stabilisierungsring an seiner Solldehnungsstelle gedehnt wird und sich aufweitet. So kann die Herzklappenprothese in radialer Richtung auseinander gedrückt werden, so dass im Inneren Raum für die neue Herzklappenprothese geschaffen wird, welche dann im Inneren der bestehenden Herzklappenprothese, insbesondere kraft- und/oder formschlüssig, fixiert wird. Die Klappensegel der alten Herzklappenprothese werden dabei ebenfalls nach außen an die Gefäßwandung gedrückt. Die Aufweitung des Stabilisierungsringes ermöglicht es, eine neue Herzklappenprothese einzusetzen, ohne dass der Innenquerschnitt wesentlich verkleinert wird. Vielmehr können durch die neue Herzklappenprothese dann sowohl der Stabilisierungsring als auch das umgebende Gewebe des Blutgefäßes so gedehnt werden, dass wieder ein angemessener Strömungsquerschnitt erreicht wird.

Bevorzugt ist die Solldehnungsstelle derart ausgebildet, dass zum Aufweiten des Stabilisierungsringes bei der Krafteinwirkung eine vorbestimmte Grenzkraft überschritten werden muss. Diese Grenzkraft ist vorzugsweise so gewählt, dass beim normalen Einsatz und insbesondere beim Implantieren der Herzklappenprothese diese Grenzkraft nicht überschritten wird, so dass der Stabilisierungsring unbeschädigt, das heißt ungedehnt in das Blutgefäß eingesetzt und dort in der oben beschriebenen Weise fixiert werden kann. Die Grenzkraft ist bevorzugt so gewählt, dass sie erst beim Einsetzen einer neuen Herzklappenprothese mit Hilfe eines Katheters erreicht wird. Dabei wird dann eine so große Kraft von Innen aufgebracht, dass die Grenzkraft überschritten wird und die Solldehnungsstelle sich dehnt, so dass der Stabilisierungsring in Umfangsrichtung erweitert wird.

Gemäß einer weiteren bevorzugten Ausführungsform ist der Stabilisierungsring aus Metall gefertigt, insbesondere aus Nitinol, Titan oder einer sonstigen geeigneten Legierung. Alternativ könnte der Stabilisierungsring auch aus einem geeigneten Kunststoff gefertigt sein.

Der Stabilisierungsring weist vorzugsweise eine derartige Festigkeit auf, dass er einer radial von außen auf ihn wirkenden Kraft, welche durch das umgebende Blutgefäß bei dessen Dehnung aufgebracht wird, widersteht, ohne das es zu einer plastischen Verformung des Stabilisierungsringes kommt. Bevorzugt widersteht der Stabilisierungsring einer größeren radialen Druckkraft von außen als von innen.

Gemäß einer besonders bevorzugten Ausführungsform kann die zumindest eine Solldehnungsstelle als eine Sollbruchstelle ausgebildet sein, an welcher der Stabilisierungsring bei der Krafteinwirkung auf seinen Innenumfang definiert reißt. Das heißt an der Solldehnungsstelle wird der Stabilisierungsring bei ausreichender Krafteinwirkung definiert getrennt, so dass er sich aufweiten kann.

Anstelle einer Sollbruchstelle kann die Solldehnungsstelle auch als Trennstelle ausgebildet sein, an welcher in Umfangsrichtung gegenüberliegende Teile des Stabilisierungsringes so aneinander anliegen, dass der Stabilisierungsring einer Druckkraft von außen widerstehen kann, sich aber bei einer Krafteinwirkung von innen in Umfangsrichtung dadurch aufweitet, dass sich die Trennstelle erweitert.

Gemäß einer weiteren bevorzugten Ausführungsform ist die Solldehnungsstelle derart ausgestaltet, dass sie bei der Krafteinwirkung plastisch in Umfangsrichtung des Stabilisierungsringes gedehnt wird. So wird die Aufweitung des Stabilisierungsringes an der Solldehnungsstelle irreversibel. Das heißt der Stabilisierungsring hält nach Wegfall der Krafteinwirkung seine aufgeweitete Form. Eine solche Solldehnungsstelle ist vorzugsweise so ausgebildet, dass sie nicht vollständig zerreißt, so dass der Stabilisierungsring als geschlossener Ring erhalten bleibt.

Erfindungsgemäß ist zumindest eine Solldehnungsstelle bzw. eine Sollbruchstelle in dem Stabilisierungsring vorgesehen. Gemäß einer besonders bevorzugten Ausführungsform, können jedoch mehrere Solldehnungs- bzw. Sollbruchstellen vorgesehen sein, wobei diese vorzugsweise gleichmäßig über den Umfang verteilt sind. Besonders bevorzugt sind drei Solldehnungsstellen vorgesehen, wobei die Solldehnungsstellen vorzugsweise im Wesentlichen an den Winkelpositionen der Kommissuren der Herzklappenprothese gelegen sind. Auf diese Weise kann eine gleichmäßige Aufweitung unter Krafteinwirkung erreicht werden, wobei sich im Bereich der Kommissuren auch das Klappengestell vorzugsweise so verformen kann, dass es die Aufweitung mitmacht.

Ferner ist zu verstehen, dass in dem Stabilisierungsring auch Kombinationen von Solldehnungsstellen, welche eine plastische Verformung ermöglichen, Sollbruchstellen und/oder Trennstellen Verwendung finden können, um die gewünschten Eigenschaften des Stabilisierungsringes, nämlich eine Widerstandsfähigkeit gegenüber Druckkräften von außen und eine Dehnbarkeit bei Krafteinwirkung von innen zu ermöglichen.

Gemäß einer weiteren bevorzugten Ausführungsform ist der Stabilisierungsring im Inneren einer Umhüllung angeordnet. Eine solche Umhüllung kann beispielsweise aus einem Gewebematerial ausgebildet sein, welches auch das Klappengestell umhüllt. Ein solches Material kann die Verbindung zu einem Nahtring herstellen oder gleichzeitig als Nahtring dienen, durch welchen ein Faden zum Vernähen geführt wird.

Die Umhüllung ist vorzugsweise so ausgebildet, dass sie sich bei Aufweitung des Stabilisierungsringes entsprechend, das heißt um dasselbe Maß wie der Stabilisierungsring, dehnt. So behindert die Umhüllung nicht die Aufweitung des Stabilisierungsringes.

Alternativ kann die Umhüllung derart ausgebildet sein, dass sie bei Aufweitung des Stabilisierungsringes, vorzugsweise an zumindest einer Sollbruchstelle zerreißt. Auch so wird sichergestellt, dass die Umhüllung die Aufweitung des Stabilisierungsringes nicht behindert. Um das Zerreißen zu ermöglichen, ist das Material der Umhüllung so gewählt, dass es bei einem Überschreiten einer Grenzkraft durch die in Umfangsrichtung wirkenden Zugkräfte an zumindest einer Sollbruchstelle definiert oder an einer beliebigen Stelle zerreißt, wenn das gesamte Material so gewählt ist, dass es bei einer Grenzkraft reißt. Die Anordnung einer Sollbruchstelle oder mehrerer Sollbruchstellen hat den Vorteil eines definierten Zerreißens, wodurch sichergestellt wird, dass keine losen Teile der Umhüllung zurückbleiben.

Erfindungsgemäß ist der Stabilisierungsring kraftentkoppelt mit dem Klappengestell verbunden. Dies ermöglicht es, dass das Klappengestell sich relativ zu dem Stabilisierungsring bewegen kann und gegenüber dem Stabilisierungsring aufweiten kann. So können sich die Bögen des Klappengestells in die Sinus aortae hinein erstrecken, wie es aus DE 10 2010 051 632 bekannt ist. Auf diese Weise wird ein maximaler Strömungsquerschnitt erreicht. Ferner kann sich so das Klappengestell beim Einsetzen einer neuen Herzklappenprothese unabhängig von dem Stabilisierungsring aufweiten, um Raum für die neue Herzklappenprothese zu schaffen. Beim Einsetzen einer neuen Herzklappenprothese weitet sich der Stabilisierungsring aufgrund der beschriebenen Sollbruchstellen bzw. Solldehnungsstellen, während sich das kraftentkoppelte Klappengestell vorzugsweise unabhängig von Sollbruch- bzw. Solldehnungsstellen in dem Stabilisierungsring allein aufgrund seiner Elastizität und/oder plastischen Verformbarkeit in radialer Richtung aufweitet.

Das Klappengestell ist elastisch verformbar ausgebildet. Dies unterstützt zum einen die Öffnungs- und Schließbewegung der Klappensegel und ermöglicht zum anderen, dass das Klappengestell zum Einsetzen in seinem Durchmesser bzw. Querschnitt verkleinert werden kann und sich anschließend aufweiten kann, insbesondere, wie vorangehend beschrieben, in die Sinus aortae. Ferner kann sich, wie vorangehend beschrieben, bei der Aufweitung des Stabilisierungsringes auch das Klappengestell noch um ein weiteres Maß aufweiten, um im Inneren Raum für eine neue Herzklappenprothese zu schaffen.

Nachfolgend wird die Erfindung beispielhaft anhand der beigefügten Figuren beschrieben. In diesen zeigt:
- Fig. 1: eine perspektivische Ansicht einer erfindungsgemäßen Herzklappenprothese,
- Fig. 2: eine Draufsicht auf die Herzklappenprothese gemäß Fig. 1 von unten,
- Fig. 3: eine perspektivische Ansicht des Klappengestells und des Stabilisierungsringes der Herzklappenprothese gemäß Fig. 1 und 2,
- Fig. 4: eine Seitenansicht des Klappengestells und des Stabilisierungsringes gemäß Fig. 3,
- Fig. 5: eine Draufsicht auf die Anordnung des Klappengestells und des Stabilisierungsringes gemäß Fig. 4 von oben,
- Fig. 6: eine perspektivische Detailansicht eines erfindungsgemäßen Stabilisierungsringes,
- Fig. 7: eine zweite Ausführungsform eines erfindungsgemäßen Stabilisierungsringes,
- Fig. 8: eine dritte Ausführungsform eines erfindungsgemäßen Stabilisierungsringes
- Fig. 9: eine vierte Ausführungsform eines erfindungsgemäßen Stabilisierungsringes.

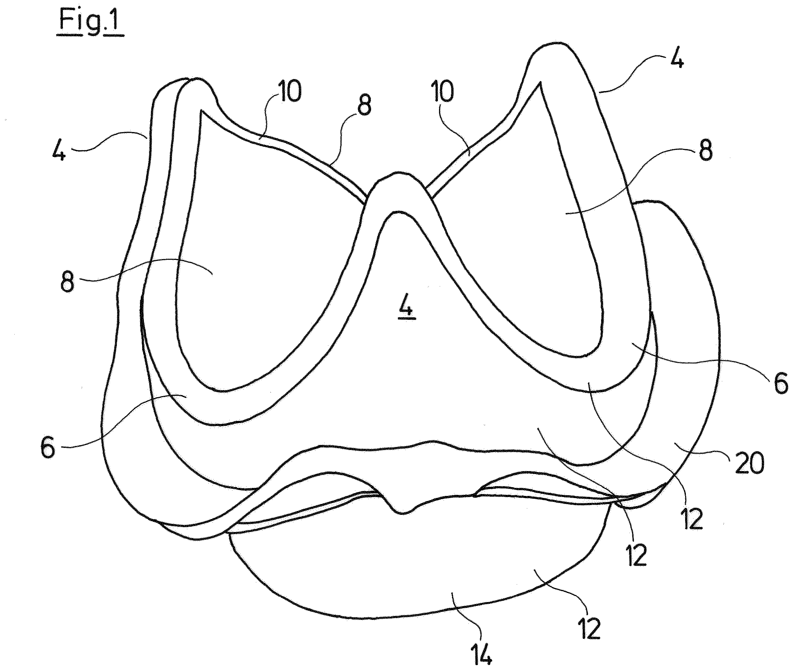

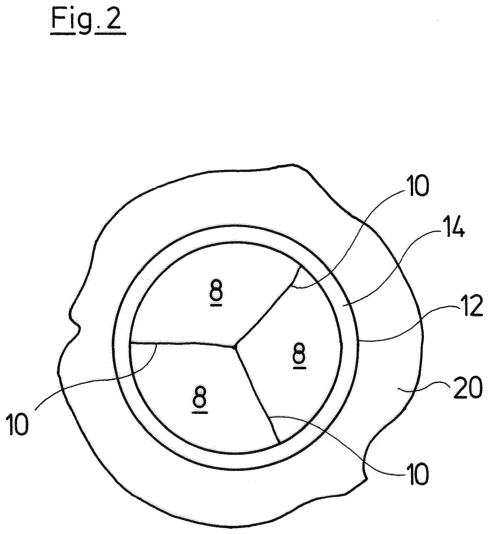

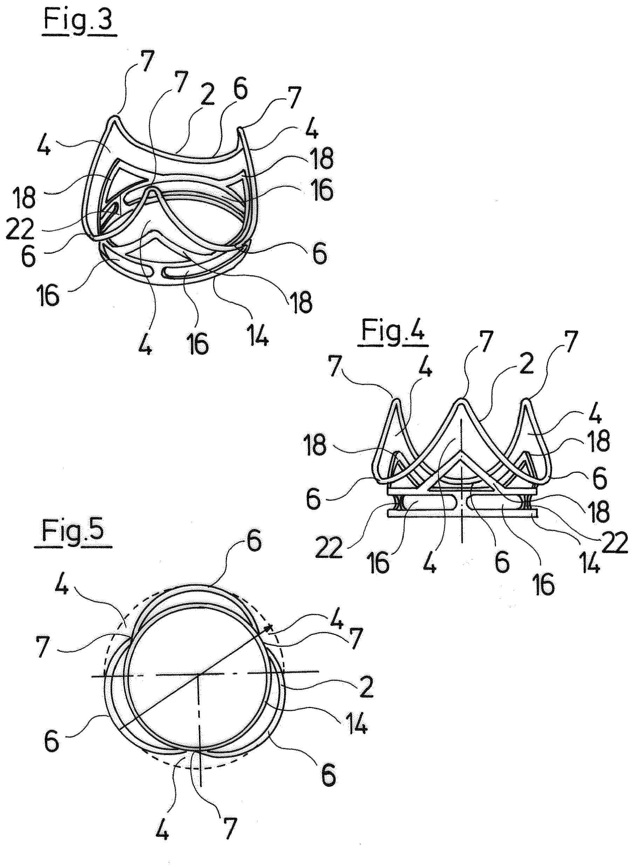

Die erfindungsgemäße Herzklappenprothese, welche in Fig. 1 und 2 gezeigt ist, weist in ihrem Inneren, wie in den Fig. 3 bis 5 gezeigt ist, ein Klappengestell 2 auf, welches drei Bögen 6 aufweist, welche an ihren Enden durch Verbindungsbögen 7 miteinander verbunden sind und so drei Kommissuren 4 bilden. Das Klappengestell 2 ist aus einem metallischen Draht geformt, welcher elastisch verformbar ist. Die drei Bögen 6 tragen drei Klappensegel 8 aus biologischem Material. Diese Taschenklappen bzw. Klappensegel 8 stoßen an drei Anlagekanten 10 aneinander und dichten dort gegeneinander ab. Zum Öffnen bewegen sich die Klappensegel 8 radial nach außen, sodass sie an den Anlagekanten 10 außer Anlage treten und eine zentrale Öffnung freigeben.

Das Klappengestell 2 ist im Übrigen von einer Umhüllung 12 aus einem Textil- bzw. Gewebematerial umhüllt, welches sich auch über die Kommissuren 4 erstreckt und diese umfänglich verschließt. Ein erstes stromabwärtiges Axialende der Herzklappenprothese wird von den Spitzen bzw. Verbindungsbögen 7 der Kommissuren 4 definiert. Am entgegengesetzten stromaufwärtigen Axialende ist ein Befestigungsbereich ausgebildet. Dort befindet sich im Inneren der Umhüllung 12 ein Stabilisierungsring 14. Der Stabilisierungsring 14 ist aus Metall gefertigt und weist eine definierte Kreisringform auf. Der Innenquerschnitt des Stabilisierungsringes 14 entspricht im Wesentlichen dem Innenquerschnitt zwischen den Spitzen der Kommissuren 14 und definiert den maximalen Strömungsquerschnitt durch die Herzklappenprothese. Dies ist insbesondere in Fig. 5 zu erkennen.

Gegenüber dem Klappengestell 2 ist der Stabilisierungsring 14 starr ausgebildet, das heißt er weist eine geringere Elastizität bzw. elastische Verformbarkeit als das Klappengestell 2 auf. Insbesondere ist der Stabilisierungsring 14 in radialer Richtung im Wesentlichen nicht elastisch verformbar. Der Stabilisierungsring 14 weist in radialer Richtung Durchbrechungen 16 auf. Diese Durchbrechungen 16 ermöglichen es, Nahtmaterial bzw. Fäden durch den Stabilisierungsring 14 zu führen, um die Herzklappenprothese an dem Stabilisierungsring 14 in einem Blutgefäß zu befestigen. Ferner weist der Stabilisierungsring 14 an einem Axialende drei sich in die Kommissuren 4 des Klappengestells 2 hinein erstreckende Kommissurenstützen 18 auf. Diese Kommissurenstützen 18 stützen im Bereich der Kommissuren 4 die Umhüllung 12, sodass diese in definierter Form gehalten wird.

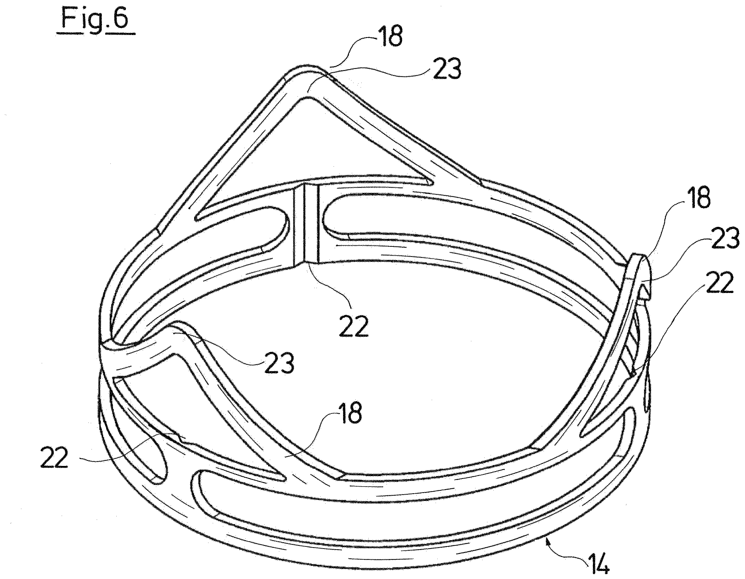

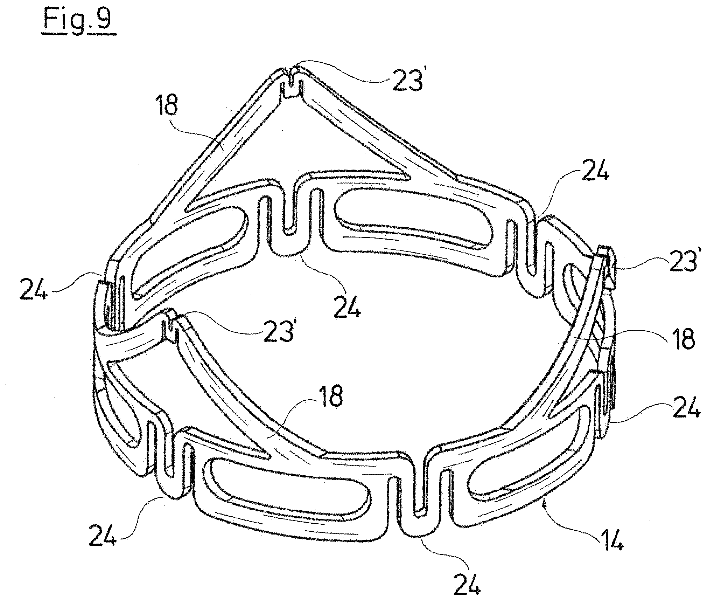

Wie insbesondere in Fig. 6 zu erkennen, weist der Stabilisierungsring 14 ferner an den Winkelpositionen der Kommissurenstützen 18 an der Innenseite sich axial erstreckende Einkerbungen 22 auf. Die Einkerbungen 22 erstrecken sich parallel zur Strömungsrichtung durch die Herzklappenprothese und bilden Sollbruchstellen. Wenn vom Innenumfang her auf den Stabilisierungsring 14 eine ausreichend große Kraft ausgeübt wird, zerreißt bzw. bricht der Stabilisierungsring im Bereich der Einkerbungen 22, das heißt die dort gebildeten Sollbruchstellen brechen und der Stabilisierungsring 14 kann sich in Umfangsrichtung aufweiten, so dass er einen größeren Durchmesser erhält. Dabei kommt es zu einer Verformung der Kommissurenstützen 18, welche dazu an ihren Spitzen verformbar mit Sollverformungs- bzw. Solldehnungsstellen 23 ausgebildet sind. Diese können alternativ auch in der in Fig. 9 gezeigten Weise ausgestaltet sein. Alternativ könnten auch im Bereich der Kommissurenstützen 18 an deren Spitzen Sollbruchstellen vorgesehen sein. Gleichzeitig ist die Umhüllung 12 so ausgebildet, dass sie sich mit dem Stabilisierungsring 14 ausdehnen kann, wenn dieser an seinen Einkerbungen 22 zerbrochen ist. Diese Ausgestaltung ermöglicht es, in das Innere dieser Herzklappenprothese eine weitere Herzklappenprothese einzusetzen und diese dann radial zu entfalten und unter Krafteinwirkung in den Innenbereich des Stabilisierungsringes 14 zu drücken, so dass dieser an seinen Einkerbungen 22 bricht und sich erweitert und gemeinsam mit dem Befestigungsbereich der neuen Herzklappenprothese gegen das umgebende Gewebe des Blutgefäßes gedrückt wird, so dass dieses ebenfalls erweitert wird. So kann die neue Herzklappenprothese unter Erweiterung der alten Herzklappenprothese in dem Blutgefäß fixiert werden, wobei der Strömungsquerschnitt im Wesentlichen nicht verkleinert wird. Dies ermöglicht mittels Katheter eine neue Herzklappenprothese einzusetzen, ohne die bisherige Herzklappenprothese entfernen zu müssen.

Es ist zu verstehen, dass anstelle einer Sollbruchstelle 22 auch eine Solldehnungsstelle ausgebildet sein kann, gemäß welcher das Material sich unter Krafteinwirkung lediglich plastisch verformt, so dass der Stabilisierungsring 14 sich in Umfangsrichtung dehnt und dadurch seinen Durchmesser erweitert. Ferner könnte auch lediglich eine Einkerbung 22 vorgesehen sein. Anstelle einer Einkerbung 22 könnte das Material auch in anderer Weise verdünnt oder geschwächt ausgebildet sein, um eine Sollbruch- oder Solldehnungsstelle zu schaffen.

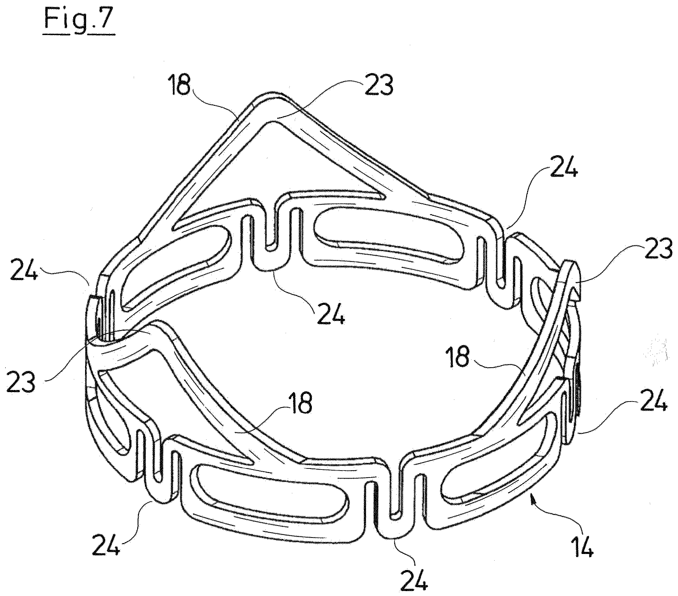

Fig. 7 zeigt einen alternativen Stabilisierungsring 14, welcher anstelle der als Sollbruchstellen fungierenden Einkerbungen 22 sechs Solldehnungsstellen 24 aufweist. Die Solldehnungsstellen 24 sind durch Biegungen bzw. Windungen des Materials quer zur Umfangsrichtung des Stabilisierungsringes 14 gebildet, sodass in den Bereichen der Solldehnungsstellen 24 das Material des Stabilisierungsringes derart plastisch verformt werden kann, dass der Stabilisierungsring 14 sich in Umfangsrichtung dehnt. Dies erfolgt durch Aufbiegung der Windungen an den Solldehnungsstellen 24. Die Kommissurenstützen 18 weisen Solldehnungsstellen 23 auf, wie sie anhand von Fig. 6 beschrieben wurden.

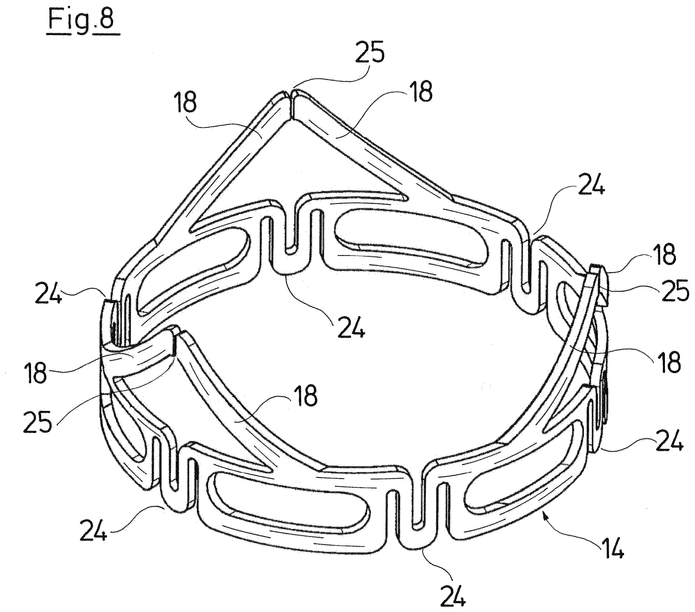

Auch bei dem Stabilisierungsring 14 gemäß Fig. 8 sind Solldehnungsstellen 24 ausgebildet, wie sie anhand von Fig. 7 beschrieben wurden. Im Unterschied zu dem Ausführungsbeispiel gemäß Fig. 7 sind an den Spitzen der Kommissurenstützen 18 keine Solldehnungsstellen 23, sondern Trennstellen 25 ausgebildet. An den Trennstellen 25 weisen die Stege, welche die Kommissurenstützen 18 bilden, eine durchgehende Fuge bzw. einen durchgehenden Spalt auf, welcher so ausgebildet ist, dass die gegenüberliegenden Seiten des Spaltes aneinander zur Anlage kommen können, um eine Druckkraft zu übertragen. Eine Zugkraft lässt sich jedoch nicht übertragen, diese führt zu einer Aufweitung des Spaltes an den Trennstellen 25, sodass sich, wenn sich die Solldehnungsstellen 24 in Umfangsrichtung dehnen, auch die Kommissurenstützen 18 durch Vergrößerung der Trennstellen 25 in Umfangsrichtung aufweiten können.

Eine weitere Variante eines Stabilisierungsringes 14 zeigt Fig. 9. Diese Variante entspricht im Wesentlichen der Variante gemäß Fig. 7, wobei die Solldehnungsstellen 23' in den Kommissurenstützen 18 auch hier durch Windungen bzw. Bögen in den die Kommissurenstützen 18 formenden Stegen ausgebildet sind. Auch diese Solldehnungsstellen 34 können sich durch Aufweitung der Bögen in Umfangsrichtung dehnen.

Die Einkerbungen 22 sowie die Solldehnungsstellen 23, 23' und 24 sind vorzugsweise so ausgebildet, dass sie erst beim Überschreiten einer vorbestimmten Grenzkraft reißen. Diese Grenzkraft ist so gewählt, dass beim Einsetzen der Herzklappenprothese in ein Blutgefäß und während ihres üblichen Einsatzes eine solche Grenzkraft nicht auf den Stabilisierungsring 14 wirkt, so dass dieser in der beschriebenen Weise formstabil ist. Nur beim Einsetzen einer neuen Herzklappenprothese durch einen in das Innere der beschriebenen Herzklappenprothese eingebrachte Katheter kann eine ausreichend große Kraft aufgebracht werden, so dass die Grenzkraft überschritten wird und die Einkerbungen 22 reißen, bzw. sich die Solldehnungsstellen 23, 23' und 24 in Umfangsrichtung dehnen.

Es ist zu verstehen, dass die beschriebenen Solldehnungsstellen 23, 23' sowie Trennstellen 25 auch zusammen mit Sollbruchstellen oder anders ausgebildeten Solldehnungsstellen Verwendung finden können. Darüber hinaus ist zu verstehen, dass anstelle der Einkerbungen 22 oder Solldehnungsstellen 24 auch in dem Stabilisierungsring 14 Trennstellen vorgesehen sein können, welche eine Aufweitung in Umfangsrichtung zulassen, jedoch dem Stabilisierungsring 14 ermöglichen, Druckkräfte, welche radial von außen auf den Stabilisierungsring 14 wirken, aufzunehmen. Ferner ist zu verstehen, dass auch andere Anzahlen von Sollbruchstellen 22 oder Solldehnungsstellen 23, 23', 24 sowie Trennstellen 25 Verwendung finden können, um einerseits eine ausreichende Stabilität des Stabilisierungsringes gegenüber Druckkräften von außen und andererseits ein Aufweiten bei Krafteinwirkung von innen zu ermöglichen.

Wie in den Fig. 3 und 4, welche die Anordnung des Klappengestells 2 und des Stabilisierungsringes 14 im Inneren der in Fig. 1 gezeigten Herzklappenprothese darstellen, zu erkennen ist, sind das Klappengestell 2 und der Stabilisierungsring 14 nicht direkt miteinander verbunden. Das Klappengestell 2 und der Stabilisierungsring 14 werden vielmehr allein durch die flexible Umhüllung 12 aus Textil- bzw. Gewebematerial miteinander verbunden, sodass eine kraftentkoppelte Verbindung zwischen dem Klappengestell 2 und dem Stabilisierungsring 14 gegeben ist. Aufgrund der Flexibilität des Gewebematerials der Umhüllung 12 kann sich das Klappengestell 2 relativ zu dem Stabilisierungsring 14 bewegen. Dies ermöglicht eine elastische Verformbarkeit des Klappengestells 2, insbesondere in radialer Richtung, während der Stabilisierungsring 14 aufgrund seiner starren Ausgestaltung beim Einsetzten in ein Blutgefäß nicht verformt wird. Durch die Umhüllung 12 bilden der Stabilisierungsring 14 und das Klappengestell 2 mit den darin ausgebildeten Klappensegeln 8 dennoch eine vorgefertigte Baueinheit, welche als Ganzes in ein Blutgefäß eingesetzt werden kann, ohne dass bei der Operation noch Montagetätigkeiten oder ein Vernähen zwischen Stabilisierungsring 14 und dem Klappengestell 2 mit dem Klappensegel 8 erforderlich wäre.

Die Fig. 3 bis 5 zeigen den unverformten entspannten Zustand des Klappengestells 2. Es ist zu erkennen, dass sich die Bögen 6 des Klappengestells 2 in diesem Zustand radial über den Außenumfang des Stabilisierungsrings 14 hinaus erstrecken, während die Kommissuren 4 im Wesentlichen auf dem Umfang des Stabilisierungsringes 14 gelegen sind. So können sich die Bögen 6 des Klappengestells 2 im implantierten Zustand in die Sinus aortae hinein erstrecken und so einen maximalen Innenquerschnitt freigeben, welcher im Wesentlichen dem natürlichen Querschnitt der Aorta entspricht. Dazu wird die gezeigte Herzklappenprothese in ihrer Dimension so auf das jeweilige Blutgefäß abgestimmt, dass der Innendurchmesser des Stabilisierungsrings 14 im Wesentlichen dem natürlichen Innendurchmesser der Aorta entspricht oder geringfügig größer ist. So kann eine gewisse Aufweitung des Gewebes des Blutgefäßes beim Einsetzen erreicht werden, sodass insgesamt der Strömungsquerschnitt durch diese Herzklappenprothese nicht eingeschränkt wird. Dazu ist der Stabilisierungsring 14 bevorzugt so ausgebildet, dass er radiale Druckkräfte von außen her aufnehmen kann, ohne dass es zu einem Brechen bzw. Reißen der von den Einkerbungen 22 gebildeten Sollbruchstellen kommt. Diese Sollbruchstellen sind somit so ausgebildet, dass sie größere Druckkräfte als Zugkräfte aufnehmen können.

Zum Einsetzen kann das Klappengestell 2, wie aus DE 10 2010 051 622 B4 bekannt ist, radial nach innen bewegt werden, sodass die Bögen 6 radial nach innen verformt werden und der maximale Außendurchmesser durch den Stabilisierungsring 14 definiert wird. So wird das Vernähen erleichtert. Nach dem Vernähen kann diese Verformung des Klappengestells 2 gelöst werden, sodass die Bögen 6 sich dann, wie vorangehend beschrieben, aufgrund ihrer Elastizität wieder erweitern und insbesondere in die Sinus aortae hinein erweitern können. Dies ist insbesondere aufgrund der flexiblen, kraftentkoppelten Anbindung des Klappengestells 2 an dem Stabilisierungsring 14 möglich. Der Stabilisierungsring 14 ist ausreichend starr ausgebildet, um den Innenquerschnitt der Herzklappenprothese zu definieren und fest in das Blutgefäß, das heißt die Aorta eingesetzt und dort fixiert zu werden. Dabei gibt der Stabilisierungsring 14 eine definierte Größe und Form der Herzklappenprothese und insbesondere deren Strömungsquerschnitts vor.

Der Stabilisierungsring 14 kann aus einer Formgedächtnislegierung, wie Nitinol, gefertigt sein, sodass es möglich ist, ihn vor dem Einsetzen radial so zu verformen, dass sein Außenquerschnitt verkleinert wird. Nach dem Einsetzen kann er sich dann durch Temperaturänderung in die gezeigte Ausgangsform zurück bewegen und dann fest in einem Blutgefäß fixiert werden, wo er dann als starre Struktur den gewünschten Querschnitt definiert.

An der Umhüllung 12 ist ferner am Außenumfang ein Nahtring 20 aus Textil- bzw. Gewebematerial angeordnet, welcher den Stabilisierungsring 14 außenumfänglich überlappt. Dies ermöglicht es, die Herzklappenprothese so in das Blutgefäß einzunähen, dass das Gewebe des Blutgefäßes zwischen dem Stabilisierungsring 14 und dem Nahtring 20 zu liegen kommt und die Fäden zum Vernähen durch den Nahtring 20, das zwischenliegende Körpergewebe und dann durch den Stabilisierungsring 14 geführt werden können. Dabei wird das Nahtmaterial durch die Umhüllung, welche den Stabilisierungsring 14 umgibt und die Durchbrechungen 16 in dem Stabilisierungsring 14 geführt. Der Stabilisierungsring 14 bildet somit ein Gegenlager beim Vernähen, sodass zusätzliche Filze als Gegenlager nicht erforderlich sind.

### Bezugszeichenliste

- 2: - Klappengestell
- 4: - Kommissuren
- 6: - Bögen
- 7: - Verbindungsbögen
- 8: - Klappensegel
- 10: - Anlagekante
- 12: - Umhüllung
- 14: Stabilisierungsring
- 16: - Durchbrechungen
- 18: - Kommissurenstützen
- 20: - Nahtring
- 22: Einkerbungen/ Sollbruchstellen
- 23, 23`, 24: Solldehnungsstellen
- 25: Trennstellen

## Patentansprüche

1. Herzklappenprothese mit einem elastisch verformbar ausgebildeten Klappengestell (2), an welchem drei Klappensegel (8) befestigt sind, wobei das Klappengestell (2) mit drei bogenförmigen Halteelementen geformt ist, welche in drei Kommissuren auslaufen und die Klappensegel (8) tragen, sowie einem sich axial an das Klappengestell (2) anschließenden Befestigungsbereich zur Befestigung in einem Blutgefäß, wobei in dem Befestigungsbereich ein Stabilisierungsring (14) angeordnet ist, welcher eine vorgegebenen Form und einen vorgegebenen Durchmesser des Befestigungsbereiches definiert, und wobei der Stabilisierungsring (14) zumindest eine Solldehnungsstelle (23, 23', 24) aufweist, welches es ermöglicht, den Stabilisierungsring (14) durch eine radiale Krafteinwirkung auf seinen Innenumfang aufzuweiten,
**gekennzeichnet dadurch, dass** der Stabilisierungsring (14) derart kraftentkoppelt mit dem Klappengestell (2) verbunden ist, dass sich das Klappengestell relativ zu dem Stabilisierungsring bewegen und aufweiten kann, sodass sich die Bögen des Klappengestells (2) in die Sinus aortae hineinstrecken können.

2. Herzklappenprothese nach Anspruch 1, bei welcher die Solldehnungsstelle (23, 23', 24) derart ausgebildet ist, dass zum Aufweiten des Stabilisierungsringes (14) bei der Krafteinwirkung eine vorbestimmte Grenzkraft überschritten werden muss.

3. Herzklappenprothese nach Anspruch 1 oder 2, bei welcher der Stabilisierungsring (14) aus Metall gefertigt ist.

4. Herzklappenprothese nach einem der vorangehenden Ansprüche, bei welcher die zumindest eine Solldehnungsstelle (23, 23', 24) als eine Sollbruchstelle (22) ausgebildet ist, an welcher der Stabilisierungsring (14) bei der Krafteinwirkung auf seinen Innenumfang definiert reißt.

5. Herzklappenprothese nach einem der Ansprüche 1 bis 3, bei welcher die Solldehnungsstelle (23, 23', 24) derart ausgestaltet ist, dass sie bei der Krafteinwirkung plastisch in Umfangsrichtung des Stabilisierungsringes (14) gedehnt wird.

6. Herzklappenprothese nach einem der vorangehenden Ansprüche, bei welcher der Stabilisierungsring (14) im Inneren einer Umhüllung (12) angeordnet ist.

7. Herzklappenprothese nach Anspruch 6, bei welcher die Umhüllung (12) derart dehnbar ist, dass sie sich bei Aufweitung des Stabilisierungsringes (14) entsprechend dehnt.

8. Herzklappenprothese nach Anspruch 6, bei welcher die Umhüllung (12) derart ausgebildet ist, dass sie bei Aufweitung des Stabilisierungsringes (14), vorzugsweise an zumindest einer Sollbruchstelle, zerreißt.

## Claims

1. Prosthetic heart valve with a valve frame (2) which is designed to be elastically deformable, on which three valve leaflets (8) are fixed, wherein the valve frame (2) is formed with three arcuate retaining elements which taper into three commissures and support the valvular leaflets (8), and a fastening area which axially adjoins the valve frame (2) for fastening in a blood vessel, wherein a stabilising ring (14) is arranged in the fastening area and defines a predetermined shape and a predetermined diameter of the fastening area,
and wherein the stabilising ring (14) has at least one predetermined expansion point (23, 23', 24), whereby it is possible to expand the stabilising ring (14) by the application of a radial force on the inner circumference thereof,
**characterized in that** the stabilising ring (14) is connected to the valve frame (2) in force decoupled manner such that the valve frame is able to move relative to the stabilising ring and can expand so that the arches of the valve frame (2) can extend into the sinus aortae.

2. Prosthetic heart valve according to Claim 1, in which the predetermined expansion point (23, 23', 24) is designed such that in order to expand the stabilising ring (14) a predetermined limit force must be exceeded by the application of the force.

3. Prosthetic heart valve according to Claim 1 or 2, in which the stabilising ring (14) is made of metal.

4. Prosthetic heart valve according to any one of the preceding claims, in which the at least one predetermined expansion point (23, 23', 24) is designed as a predetermined breaking point (22), at which the stabilising ring (14) ruptures with the application of force to the inner circumference thereof.

5. Prosthetic heart valve according to any one of Claims 1 to 3, in which the predetermined expansion point (23, 23', 24) is designed such that it expands plastically in the circumferential direction of the stabilising ring (14) with the application of force.

6. Prosthetic heart valve according to any one of the preceding claims, in which the stabilising ring (14) is arranged inside a casing (12).

7. Prosthetic heart valve according to Claim 6, in which the casing (12) is able to stretch in such manner that it stretches correspondingly with the expansion of the stabilising ring (14).

8. Prosthetic heart valve according to Claim 6, in which the casing (12) is designed in such manner that it ruptures, preferably at at least one predetermined breaking point when the stabilising ring (14) expands.

## Revendications

1. Prothèse de valve cardiaque comportant une structure de valve (2) façonnée pour être déformable par élasticité, sur laquelle sont fixés trois feuillets de valve (8), la structure de valve (2) étant formée avec trois éléments de maintien en forme d'arc qui se terminent en trois commissures et supportent les feuillets de valve (8), ainsi qu'une zone de fixation raccordée de manière axiale à la structure de valve (2) et destinée à la fixation dans un vaisseau sanguin, un anneau de stabilisation (14) étant agencé dans la zone de fixation, cet anneau définissant une forme et un diamètre prédéterminés de la zone de fixation,
et dans laquelle l'anneau de stabilisation (14) présente au moins un point d'extension théorique (23, 23', 24) qui permet d'élargir l'anneau de stabilisation (14) par une application de force radiale sur sa périphérie intérieure,
**caractérisée en ce que** l'anneau de stabilisation (14) est relié à la structure de valve (2) avec désaccouplement de force, de telle sorte que la structure de valve peut se déplacer et s'élargir par rapport à l'anneau de stabilisation, de telle manière que les arcs de la structure de valve (2) puissent s'étendre dans les sinus aortiques.

2. Prothèse de valve cardiaque selon la revendication 1, dans laquelle le point d'extension théorique (23, 23', 24) est conçu de manière telle que, lors de l'application de force, une force limite prédéterminée doit être dépassée pour élargir l'anneau de stabilisation (14).

3. Prothèse de valve cardiaque selon la revendication 1 ou 2, dans laquelle l'anneau de stabilisation (14) est réalisé en métal.

4. Prothèse de valve cardiaque selon l'une quelconque des revendications précédentes, dans laquelle le point d'extension théorique (23, 23', 24), au moins au nombre de un, est conçu sous la forme d'un point de rupture théorique (22) au niveau duquel l'anneau de stabilisation (14) se rompt de manière définie lors de l'application de force sur sa périphérie intérieure.

5. Prothèse de valve cardiaque selon l'une quelconque des revendications 1 à 3, dans laquelle le point d'extension théorique (23, 23', 24) est conçu de manière à être étiré de manière plastique dans la direction circonférentielle de l'anneau de stabilisation (14) lors de l'application de force.

6. Prothèse de valve cardiaque selon l'une quelconque des revendications précédentes, dans laquelle l'anneau de stabilisation (14) est agencé à l'intérieur d'une enveloppe (12).

7. Prothèse de valve cardiaque selon la revendication 6, dans laquelle l'enveloppe (12) est extensible de manière telle qu'elle s'étire de manière correspondante lors de l'élargissement de l'anneau de stabilisation (14).

8. Prothèse de valve cardiaque selon la revendication 6, dans laquelle l'enveloppe (12) est conçue de manière à se déchirer lors de l'élargissement de l'anneau de stabilisation (14), de préférence au niveau d'au moins un point de rupture théorique.
